# EUROPEAN PATENT APPLICATION

(11) **EP 4 850 609 A1**
(43) Date of publication of application: **09.12.2026**
(21) Application number: 25181125.3
(22) Date of filing: 05.06.2025
(51) Int. Cl.: A61B 8/00, A61B 8/08

(54) **METHODS AND SYSTEMS FOR AUTOMATED REAL-TIME SCORING OF ULTRASOUND IMAGE QUALITY**

(71) Applicant: Koninklijke Philips N.V., 5656 AE Eindhoven (NL)
(72) Inventor: CHEN, Alvin, Eindhoven (NL); BALARAJU, Naveen, Eindhoven (NL); PATIL, Shubham, 5656AG Eindhoven (NL)
(74) Representative: Philips Intellectual Property & Standards

(57) **Abstract**

A method (100) for analyzing quality of ultrasound imaging, comprising: receiving (120) ultrasound imaging comprising a plurality of ultrasound frames per second; analyzing (130) the received ultrasound imaging with an anatomical landmark detection algorithm to generate a detector-based quality score comprising a likelihood of the anatomical landmark's presence in the received ultrasound imaging; analyzing (140) the received ultrasound imaging with a trained frame score classifier to generate a classifier-based quality score for each of the plurality of ultrasound frames; generating (150), by combining the detector-based quality score and the classifier-based quality score, an image view quality score for each ultrasound frame; providing (160) the generated image view quality score for one or more of the plurality of ultrasound frames; and generating (170), from the generated image view quality scores for a plurality of ultrasound frames, an ultrasound image view quality score.

## Description

### FIELD OF THE INVENTION

The present disclosure is directed generally to methods and systems for determining and providing real-time scoring of ultrasound image quality using an ultrasound imaging analysis system.

### BACKGROUND OF THE INVENTION

Ultrasound technology has become a ubiquitous and indispensable tool in modern medicine. Its widespread adoption is due to its non-invasive nature, real-time imaging capabilities, and versatility in diagnosing and monitoring a variety of medical conditions. Ultrasound exams are routinely performed for a wide variety of analytical, diagnostic, and preventative reasons.

High quality ultrasound imaging is essential for making accurate diagnostic evaluations. For example, poor image quality increases the risk of misdiagnosis. An optimal ultrasound image should clearly display key anatomical landmarks to confirm proper transducer positioning within the correct imaging view. Additionally, the image should have appropriate settings for gain, contrast, and depth, along with good acoustic coupling and minimal transducer motion. Experienced ultrasound users learn to acquire appropriate scans and make clinical decisions based on images of sufficient quality. For newer users, however, it is often difficult to recognize whether an image meets criteria for diagnostic interpretation.

### SUMMARY OF THE INVENTION

There is thus a continued need for methods and systems that determine and provide real-time scoring of ultrasound image quality.

Various embodiments and implementations are directed to methods and systems for analyzing the quality of ultrasound imaging. Ultrasound imaging of a first location of a patient is received, and an anatomical landmark detection algorithm analyzes the imaging to identify anatomical landmarks and generate a detector-based quality score comprising a likelihood of the anatomical landmark's presence in the received ultrasound imaging. The imaging is also analyzed with a trained frame score classifier to generate a classifier-based quality score for each of the plurality of ultrasound frames. The detector-based quality score and the classifier-based quality score are combined to generate an image view quality score for each ultrasound frame. The generated image view quality score is provided to a user via a user interface. The system also generates an overall ultrasound image view quality score from the quality scores for a plurality of ultrasound frames, and can provide that ultrasound image view quality score via the user interface.

According to an aspect, a method for analyzing quality of ultrasound imaging is provided. The method includes: (i) receiving ultrasound imaging obtained from a first location of a patient, the ultrasound imaging comprising a plurality of ultrasound frames per second; (ii) analyzing the received ultrasound imaging with an anatomical landmark detection algorithm to identify one or more anatomical landmarks in the received ultrasound imaging, wherein a detector-based quality score is determined based on the analysis by the anatomical landmark detection algorithm, the detector-based quality score comprising a likelihood of the anatomical landmark's presence in the received ultrasound imaging; (iii) analyzing the received ultrasound imaging with a trained frame score classifier to generate a classifier-based quality score for each of the plurality of ultrasound frames, wherein the classifier-based quality score indicates a quality of a respective ultrasound frame; (iv) generating, by combining the detector-based quality score and the classifier-based quality score, an image view quality score for each of the plurality of ultrasound frames; (v) providing, to a user via a user interface, the generated image view quality score for one or more of the plurality of ultrasound frames; and (vi) generating, from the generated image view quality scores for a plurality of ultrasound frames, an ultrasound image view quality score.

In accordance with an embodiment, the generated image view quality score is a sum or average of quality scores for each of a plurality of anatomical landmarks in the received ultrasound imaging.

In accordance with an embodiment, the generated image view quality score comprises a weight indicating an importance of the presence of the respective anatomical landmark in the received ultrasound imaging.

In accordance with an embodiment, the anatomical landmark detection algorithm is a trained machine learning model.

In accordance with an embodiment, the anatomical landmark detection algorithm and the trained frame score classifier are a single model.

In accordance with an embodiment, the classifier-based quality score is a value including or between 0 and 1.

In accordance with an embodiment, the anatomical landmark detection algorithm and the trained frame score classifier analyze the received ultrasound imaging in sequence.

In accordance with an embodiment, the anatomical landmark detection algorithm and the trained frame score classifier analyze the received ultrasound imaging in parallel.

In accordance with an embodiment, the generated image view quality score is provided via the user interface as a numerical score or as a color.

In accordance with an embodiment, the ultrasound imaging quality score is generated by a trained ultrasound imaging quality score model.

In accordance with an embodiment, the method further comprises providing, to a user via a user interface, the generated ultrasound imaging quality score.

According to another aspect is a system for analyzing quality of ultrasound imaging. The system includes: an anatomical landmark detection algorithm configured to analyze received ultrasound imaging to identify one or more anatomical landmarks in the received ultrasound imaging, wherein a detector-based quality score is determined based on the analysis by the anatomical landmark detection algorithm, the detector-based quality score comprising a likelihood of the anatomical landmark's presence in the received ultrasound imaging; a trained frame score classifier configured to analyze received ultrasound imaging to generate a classifier-based quality score for each of the plurality of ultrasound frames, wherein the classifier-based quality score indicates a quality of a respective ultrasound frame; a user interface; and a processor configured to: (i) receive ultrasound imaging obtained from a first location of a patient, the ultrasound imaging comprising a plurality of ultrasound frames per second; (ii) analyze the received ultrasound imaging with the anatomical landmark detection algorithm to generate a detector-based quality score for each of the plurality of ultrasound frames; (iii) analyze the received ultrasound imaging with the trained frame score classifier to generate a classifier-based quality score for each of the plurality of ultrasound frames; (iv) combine the detector-based quality score and the classifier-based quality score to generate an image view quality score for each of the plurality of ultrasound frames; (v) provide, via the user interface, the generated image view quality score for one or more of the plurality of ultrasound frames; and (vi) generate an ultrasound image view quality score from the generated image view quality scores for a plurality of ultrasound frames.

It should be appreciated that all combinations of the foregoing concepts and additional concepts discussed in greater detail below (provided such concepts are not mutually inconsistent) are contemplated as being part of the inventive subject matter disclosed herein. In particular, all combinations of claimed subject matter appearing at the end of this disclosure are contemplated as being part of the inventive subject matter disclosed herein. It should also be appreciated that terminology explicitly employed herein that also may appear in any disclosure incorporated by reference should be accorded a meaning most consistent with the particular concepts disclosed herein.

These and other aspects of the various embodiments will be apparent from and elucidated with reference to the embodiment(s) described hereinafter.

### BRIEF DESCRIPTION OF THE DRAWINGS

In the drawings, like reference characters generally refer to the same parts throughout the different views. The figures showing features and ways of implementing various embodiments and are not to be construed as being limiting to other possible embodiments falling within the scope of the attached claims. Also, the drawings are not necessarily to scale, emphasis instead generally being placed upon illustrating the principles of the various embodiments.
Fig. 1 is a flowchart of a method for analyzing quality of ultrasound imaging, in accordance with an embodiment.
Fig. 2 is a schematic representation of an ultrasound imaging analysis system, in accordance with an embodiment.
Fig. 3 is a flowchart of a method for analyzing quality of ultrasound imaging, in accordance with an embodiment.
Fig. 4 is a flowchart of a method for deriving a detector-based quality score from the sum of confidence scores for detected anatomical landmarks, in accordance with an embodiment.
Fig. 5 is a flowchart of a method for deriving a detector-based quality score from the sum of confidence scores for detected anatomical landmarks, in accordance with an embodiment.
Fig. 6 is a flowchart of a method for deriving a detector-based quality score, in accordance with an embodiment.
Fig. 7 is a of a method for generating a classifier-based quality score, in accordance with an embodiment.
Fig. 8 is a flowchart of a method for combining a detector-based quality score and a classifier-based quality score to generate an image view quality score, in accordance with an embodiment.
Fig. 9 is a flowchart of a method for combining a detector-based quality score and a classifier-based quality score to generate an image view quality score, in accordance with an embodiment.
Fig. 10 is a flowchart of a method for combining a detector-based quality score and a classifier-based quality score to generate an image view quality score, in accordance with an embodiment.
Fig. 11 is a flowchart of a method for combining a detector-based quality score and a classifier-based quality score to generate an image view quality score, in accordance with an embodiment.
Fig. 12 is a flowchart of a method for combining frame-level confidence scores into an overall cineloop-level quality score, in accordance with an embodiment.
Fig. 13 is a flowchart of a method for training an anatomical landmark detection algorithm, in accordance with an embodiment.

### DETAILED DESCRIPTION OF EMBODIMENTS

The present disclosure describes various embodiments of a system and method configured to analyze the quality of ultrasound imaging. More generally, Applicant has recognized and appreciated that it would be beneficial to provide an indication of ultrasound imaging quality to a user. Accordingly, ultrasound imaging of a first location of a patient is received, and an anatomical landmark detection algorithm analyzes the imaging to identify anatomical landmarks and generate a detector-based quality score comprising a likelihood of the anatomical landmark's presence in the received ultrasound imaging. The imaging is also analyzed with a trained frame score classifier to generate a classifier-based quality score for each of the plurality of ultrasound frames. The detector-based quality score and the classifier-based quality score are combined to generate an image view quality score for each ultrasound frame. The generated image view quality score is provided to a user via a user interface. The system also generates an overall ultrasound image view quality score from the generated image view quality scores for a plurality of ultrasound frames, and can provide that ultrasound imaging quality score via the user interface.

The embodiments and implementations disclosed or otherwise envisioned herein can be utilized with any system or process that may utilize or benefit from ultrasound imaging quality analysis or scoring. The embodiments and implementations disclosed or otherwise envisioned herein can be utilized with any system that generates or analyzes ultrasound imaging, including but not limited to Philips^{®} Lumify^{®} (manufactured by Koninklijke Philips, N.V.), among other products and imaging modalities. However, the disclosure is not limited to these devices or systems, and thus disclosure and embodiments disclosed herein can encompass any system that may utilize or benefit from ultrasound imaging quality analysis or scoring.

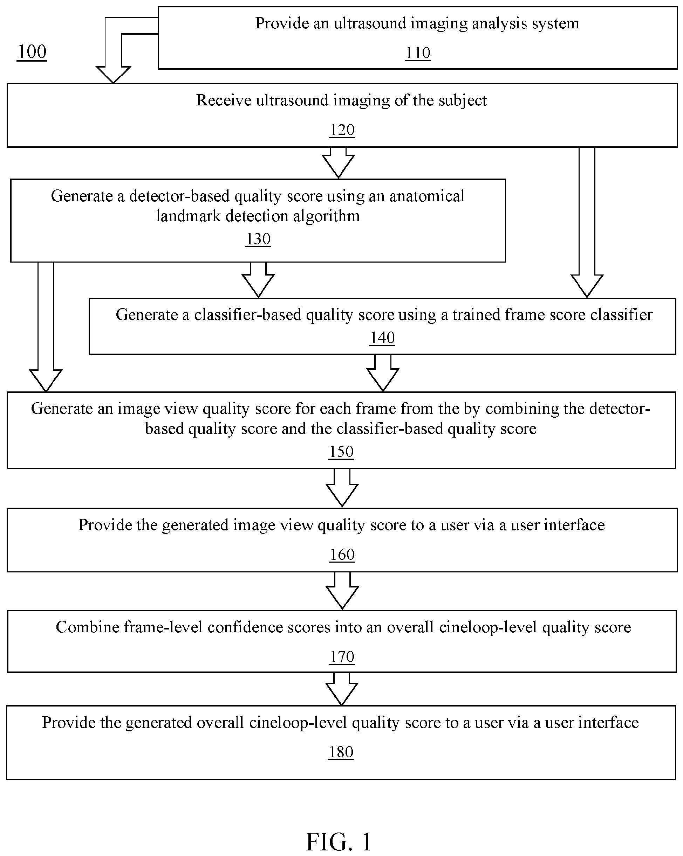

Referring to Fig. 1, in one embodiment, is a flowchart of a method for real-time scoring of ultrasound image quality using an ultrasound imaging analysis system. The methods described in connection with the figures are provided as examples only, and shall be understood not to limit the scope of the disclosure. The ultrasound imaging analysis system can be any of the systems described or otherwise envisioned herein. The ultrasound imaging analysis system can be a single system or multiple different systems.

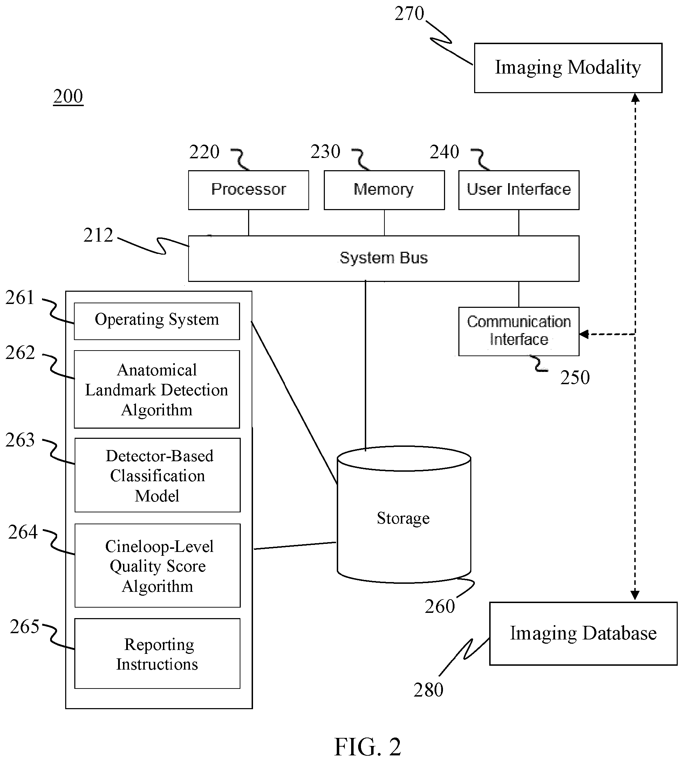

At step 110 of the method, an ultrasound imaging analysis system 200 is provided. Referring to an embodiment of an ultrasound imaging analysis system 200 as depicted in Fig. 2, for example, the system comprises one or more of a processor 220, memory 230, user interface 240, communications interface 250, and storage 260, interconnected via one or more system buses 212. It will be understood that Fig. 2 constitutes, in some respects, an abstraction and that the actual organization of the components of the system 200 may be different and more complex than illustrated. Additionally, ultrasound imaging analysis system 200 can be any of the systems described or otherwise envisioned herein. Other elements and components of the ultrasound imaging analysis system 200 are disclosed and/or envisioned elsewhere herein.

According to an embodiment, the ultrasound imaging analysis system 200 comprises or is in direct or indirect communication with an imaging modality 270. The imaging modality can be, for example, an ultrasound system such as EPIQ^{®} system, Philips Lumify^{®}, and Philips Affinity^{®}, among many others. The images obtained using the imaging modality may be obtained from a clinical provider or other individual. For example, the user of the imaging modality may be an experienced sonographer, a first-time user of ultrasound, or any experience level in between.

According to an embodiment, the ultrasound imaging analysis system 200 comprises or is in direct or indirect communication with an image database 280. The image database may be any image database, and may comprise images or videos or reports or other data obtained using any imaging modality, such as imaging modality 270. The image database 280 may be local to the ultrasound system, and may optionally be a component of the system. The image database 280 may alternatively be remote to the ultrasound system, and thus is in direct or indirection communication with the ultrasound system.

According to an embodiment, the ultrasound imaging analysis system 200 provides automated, real-time scoring of ultrasound image view quality. According to an embodiment, a quality score is generated for each frame in ultrasound imaging, with a higher score indicating better view quality and a lower score indicating poorer view quality. The score may be presented numerically (e.g., on a scale from 0 to 1) or visually (e.g., with a green indicator for good quality and a red indicator for poor quality), among other possible methods for presenting quality scoring. When a cineloop is saved, scores for individual frames are summed or averaged into a cineloop-level quality score.

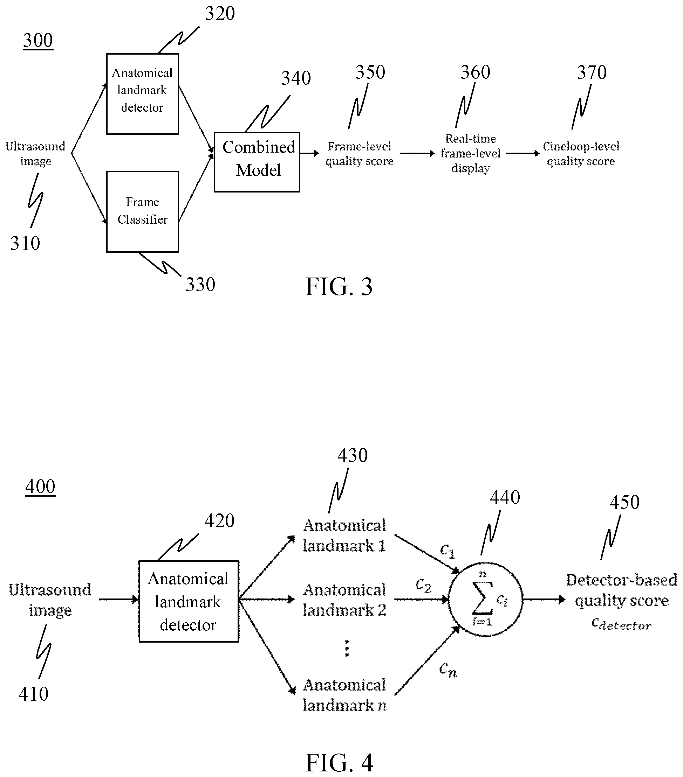

Referring to Fig. 3, in one embodiment, is a flowchart showing a high-level overview of a method 300 for real-time scoring of ultrasound image quality using an ultrasound imaging analysis system. Ultrasound imaging is obtained or received at step 310. The received ultrasound imaging is analyzed by: (1) an anatomical landmark detector at 320 to generate a detector-based quality score, and (2) a frame classifier at 330 to generate a classifier-based quality score. The detector-based quality score and the classifier-based quality score are combined at 340 to generate a frame-level image view quality score 350. The frame-level image view quality score is provided to a user via a user interface at 360. At 370, an ultrasound imaging quality score (also called a cineloop-level quality score) is generated from the detector-based quality scores for each of the plurality of ultrasound frames. Below, the method for real-time scoring of ultrasound image quality using an ultrasound imaging analysis system is described in greater detail.

At step 120 of method 100 in Fig. 1, ultrasound imaging of the patient or subject is obtained using the imaging modality 270 of the ultrasound system 200. The patient or subject can be any subject that could potentially benefit from ultrasound. According to one embodiment, the ultrasound imaging may be obtained of any region of the subject's body, such as the abdomen, chest, head, or limbs among other regions. The ultrasound imaging may be obtained using any method, system, or technique for obtaining ultrasound imaging. The ultrasound imaging comprises a plurality of frames per second. Once obtained, the ultrasound imaging may be utilized immediately, and/or it may be temporarily or permanently stored in local and/or remote memory for future use, including in image database 280.

At step 130 of the method, the ultrasound imaging analysis system 200 analyzes the received or obtained ultrasound imaging using an anatomical landmark detection algorithm. The anatomical landmark detection algorithm is configured or programmed or trained to identify one or more anatomical landmarks in the received or obtained ultrasound imaging. The anatomical landmark detection algorithm is further configured or programmed or trained to generate, based on the identification of the one or more anatomical landmarks, a detector-based quality score. The detector-based quality score comprises a likelihood of the anatomical landmark's presence in the received ultrasound imaging.

According to an embodiment, the anatomical landmark detection algorithm generates the detector-based quality score in real-time for each ultrasound frame of the ultrasound imaging, such as when it is received or obtained.

According to an embodiment, the anatomical landmark detection algorithm is configured or programmed or trained to determine presence and/or absence of critical anatomical landmarks that must be present for an image to be diagnostically usable. Clinical guidelines have established these landmarks for a wide range of ultrasound applications, including cardiac, trauma, and lung imaging. For example, in lung ultrasound, the pleural line is the key landmark. Other anatomical structures, such ribs, diaphragm, liver/spleen, and kidney, can be used to differentiate between moderate- and high-quality images.

According to one possible embodiment, the anatomical landmark detection algorithm is a trained machine learning algorithm. The anatomical landmark detection algorithm, whether or not a trained machine learning algorithm, is used to identify the presence and location of critical anatomical landmarks. The detector outputs a set of location coordinates (*xᵢ*, *yᵢ*) for each detected landmark *i*, along with confidence scores *cᵢ* that indicate the likelihood of each landmark's presence. According to an embodiment, the detected confidences *cᵢ* are summed or averaged to derive an overall detector-based confidence score (*c_{detector}*).

Referring to Fig. 4, in one embodiment, is a flowchart of a method 400 for deriving a detector-based quality score from the sum of confidence scores for detected anatomical landmarks. According to an embodiment, ultrasound imaging 410 is provided or obtained. An anatomical landmark detector (i.e., the anatomical landmark detection algorithm) 420 analyzes the ultrasound imaging and identifies one or more anatomical landmarks (*i*) at 430, and generates a confidence score (*cᵢ*) for each landmark indicating the likelihood of the landmark's presence. The detected confidences *cᵢ* are summed or averaged at 440 to generate a detector-based quality score 450.

According to an embodiment, different anatomical structures can have different or varying degrees of importance in determining image quality. Accordingly, the system can use a weighted sum or average can be used. For example, in lung ultrasound, the pleural line is critical and is given a higher weight, while other structures, such as the ribs, diaphragm, liver, spleen, and kidney, are assigned lower weights. The weights can be experimentally derived, learned during training of a machine learning algorithm, or pre-programmed.

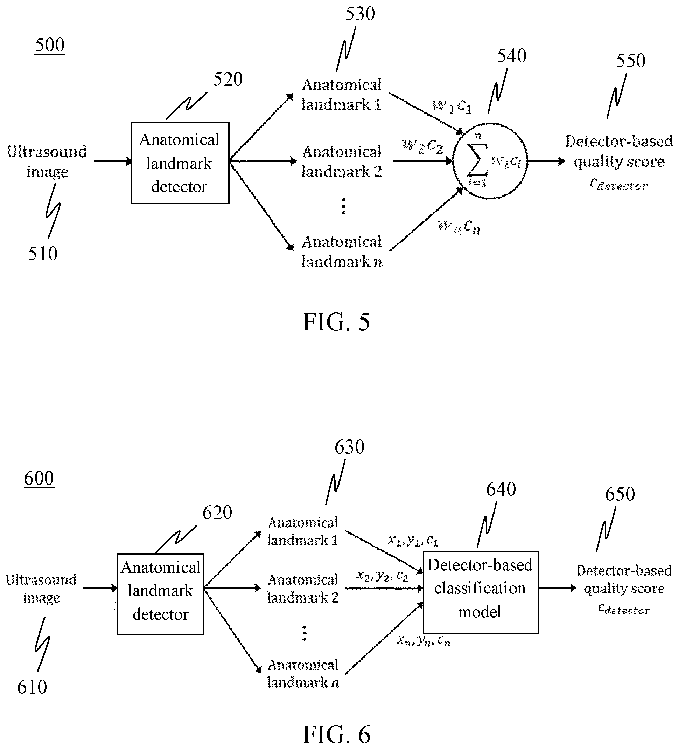

Referring to Fig. 5, in one embodiment, is a flowchart of a method 500 for deriving a detector-based quality score from the sum of confidence scores for detected anatomical landmarks. According to an embodiment, ultrasound imaging 510 is provided or obtained. An anatomical landmark detector (i.e., the anatomical landmark detection algorithm) 520 analyzes the ultrasound imaging and identifies one or more anatomical landmarks (*i*) at 530, and generates a weighted confidence score (*wᵢcᵢ*) for each landmark indicating the likelihood of the landmark's presence. The weighted confidences *wᵢcᵢ* are summed or averaged at 540 to generate a detector-based quality score 550.

According to an embodiment, the weights assigned to detected anatomical landmarks can be optimized in a data-driven manner, for example by using another algorithm such as a detector-based classification model, which may optionally be a trained machine learning classification model. According to an embodiment, the detector-based classification model may take as input only the detection confidence scores (*cᵢ*), or it may take also include the location coordinates (*xᵢ, yᵢ*) along with detection confidence scores (*cᵢ*) (i.e., (*xᵢ, yᵢ, cᵢ*))*.*

Referring to Fig. 6, in one embodiment, is a flowchart of a method 600 for deriving a detector-based quality score in which the locations (*xᵢ, yᵢ*) and confidences (*cᵢ*) of detected anatomical landmarks are combined. According to an embodiment, ultrasound imaging 610 is provided or obtained. An anatomical landmark detector (i.e., the anatomical landmark detection algorithm) 620 analyzes the ultrasound imaging and identifies one or more anatomical landmarks (*i*) at 530, identifies the location (*xᵢ, yᵢ*) of each of the one or more anatomical landmarks, and generates a confidence score (*cᵢ*) at 630 for each of the one or more anatomical landmarks indicating the likelihood of the landmark's presence. A detector-based classification model 640 uses the location coordinates (*xᵢ, yᵢ*) along with detection confidence scores (*cᵢ*) (i.e., (*xᵢ, yᵢ, cᵢ*)) at 640 to generate a detector-based quality score 650.

Once generated, the detector-based quality score may be utilized immediately, and/or it may be temporarily or permanently stored in local and/or remote memory for future use.

According to an embodiment, an advantage of determining image view quality based on detected anatomical landmarks is the method's explainability. Users can easily understand how the quality score is derived, as the detected landmarks are highlighted on the screen. A potential drawback is that other factors influencing image quality may not be captured by anatomical detection alone.

Accordingly, at step 140 of the method in Fig. 1, the ultrasound imaging analysis system 200 analyzes the received or obtained ultrasound imaging using a trained frame score classifier. The trained frame score classifier is trained to analyze the received or obtained ultrasound imaging in order to generate a classifier-based quality score. The classifier-based quality score comprises or indicates a quality of a respective ultrasound frame. According to an embodiment, the trained frame score classifier generates the classifier-based quality score in real-time for each ultrasound frame of the ultrasound imaging, such as when it is received or obtained.

According to an embodiment, in addition to inferring image view quality from the presence of anatomical landmarks, a quality score can be learned directly. In this approach, a machine learning classifier predicts the quality score based on the overall visual appearance of the image. In a preferred embodiment, the classifier is a deep convolutional neural network. As with the previous method, a high score indicates good image quality, while a low score indicates poor quality.

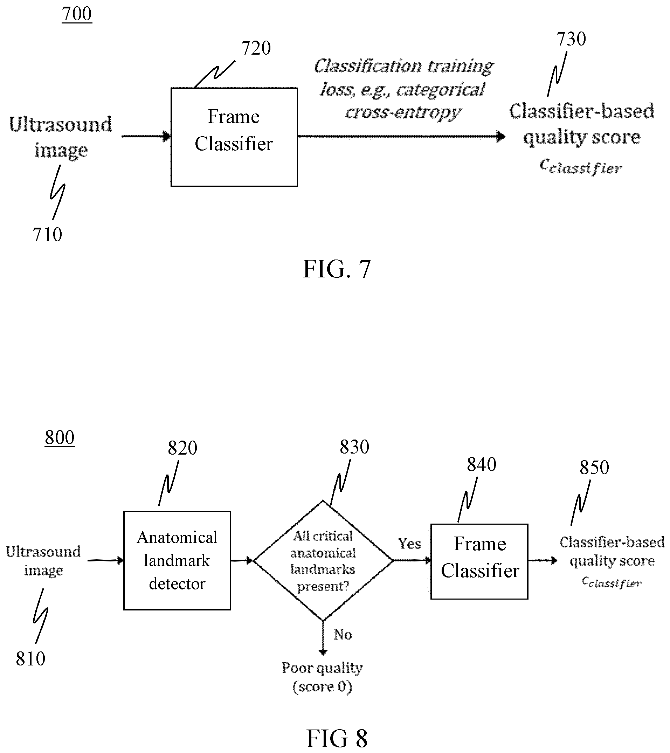

Referring to Fig. 7, in one embodiment, is a flowchart of a method 700 for generating a classifier-based quality score. According to an embodiment, ultrasound imaging 710 is provided or obtained. A trained frame classifier 720 analyzes the analyzes the ultrasound imaging and generates a classifier-based quality score 730.

Once generated, the classifier-based quality score may be utilized immediately, and/or it may be temporarily or permanently stored in local and/or remote memory for future use.

According to an embodiment, an advantage of direct classification is that the classifier can learn and incorporate factors affecting image quality beyond anatomical landmarks, such as image gain and contrast, imaging depth, noise, acoustic coupling and shadowing, probe motion, and probe angle. However, a disadvantage of direct classification is that it is less interpretable compared to determining image quality based on detected anatomical landmarks.

Accordingly, at step 150 of the method, the detector-based quality score and the classifier-based quality score are combined to generate an image view quality score for each of the plurality of ultrasound frames. According to an embodiment, the detector-based quality score and the classifier-based quality score can be combined in a variety of different ways to generate an image view quality score.

For example, referring to Fig. 8 is one embodiment of a method 800 for combining the detector-based quality score and the classifier-based quality score to generate an image view quality score. In this embodiment, the anatomical detection and frame classification are sequential. According to an embodiment, ultrasound imaging 810 is provided or obtained. The anatomical landmark detector 820 identifies critical anatomical landmarks. If all critical landmarks are detected, see step 830, the frame classifier is run in a next step 840 to provide a final quality score 850 (*c_{classifier}*) based on image appearance. If one or more critical landmarks are missing, the image is classified as poor quality (score = 0).

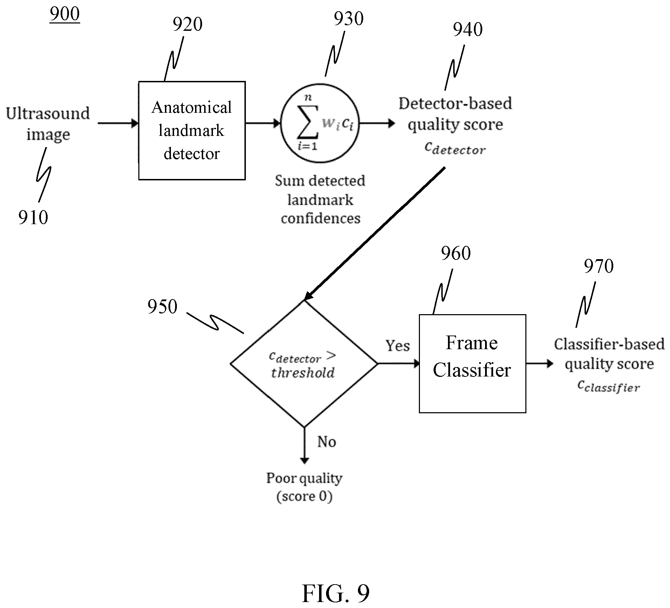

As another example, referring to Fig. 9 is another embodiment of a method 900 for combining the detector-based quality score and the classifier-based quality score to generate an image view quality score. In this embodiment, the anatomical detection and frame classification are sequential and there is a detector-based confidence score. According to an embodiment, ultrasound imaging 910 is provided or obtained. The anatomical landmark detector 920 identifies anatomical landmarks as well as the confidences *cᵢ* of the anatomical landmarks, and optionally provides the location coordinates (*xᵢyᵢ*) of the anatomical landmarks. The confidences are summed or averaged at 930 to derive a combined detector-based score (*c_{detector}*) 940. The combined detector-based score (*c_{detector}*) is compared to a predetermined threshold at 950. If the detector-based score meets or exceeds the predefined threshold, the frame classifier 960 is run as a second step to provide a final quality score 970 based on image appearance (*c_{classifier}*). Otherwise, the image is classified as poor quality.

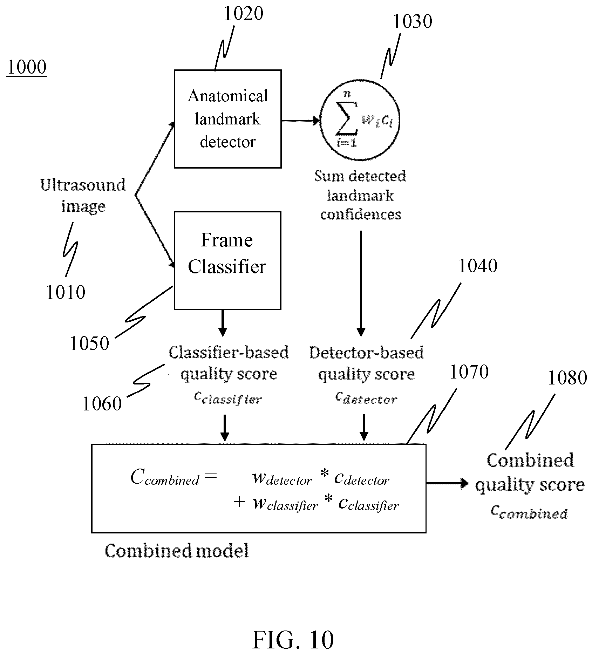

As another example, referring to Fig. 10 is another embodiment of a method 1000 for combining the detector-based quality score and the classifier-based quality score to generate an image view quality score. In this embodiment, the anatomical detection and frame classification are performed in parallel. According to an embodiment, ultrasound imaging 1010 is provided or obtained. The anatomical landmark detector 1020 identifies anatomical landmarks and provides confidences *cᵢ* of landmarks, which are combined at 1030 into a detector-based score (*c_{detector}*) 1040. The frame classifier analyzes the ultrasound imaging at 1050 in parallel and provides a separate quality score (*c_{classifier}*) at 1060 based on image appearance. The two outputs are combined at 1070 into a final score (*c_{combined}*) 1080 using a weighted sum or average.

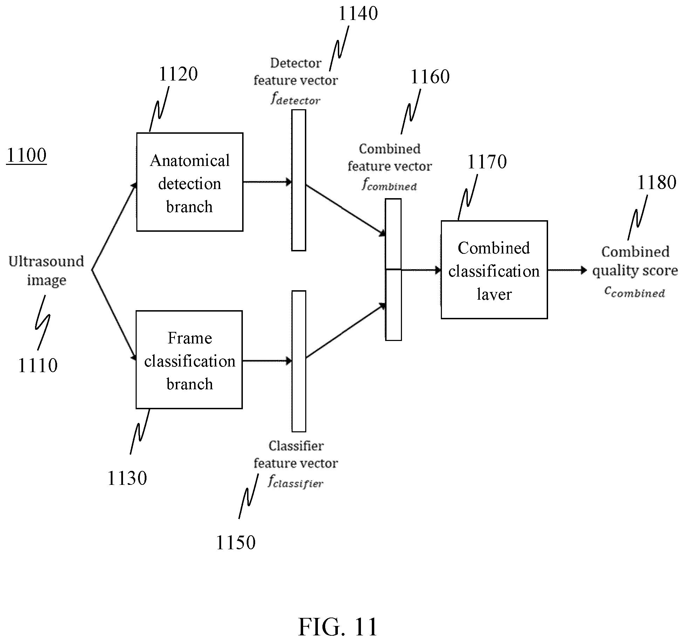

As another example, referring to Fig. 11 is another embodiment of a method 1100 for combining the detector-based quality score and the classifier-based quality score to generate an image view quality score. In this embodiment, the anatomical detection and frame classification are performed by a single trained anatomical detection and frame classification model. According to an embodiment, ultrasound imaging 1110 is provided or obtained. The anatomical landmark detector and frame classifier separate branches (1120 and 1130, respectively) of a single machine learning model, to generate one or more detector feature vectors (*f_{detector}*) at 1140, and one or more classifier feature vectors (*f_{classifier}*) at 1150. The features from the two branches are concatenated (*f_{combined}*) at 160 into a final, combined classification layer 1170 to generate a combined quality score (*c_{combined}*) *1180.*

According to an embodiment, combining the detector-based quality score and the classifier-based quality score to generate an image view quality score provides numerous advantages to the ultrasound imaging analysis system 200. For example, while determining image view quality based on detected anatomical landmarks has the advantage of being easily understood by a sonographer or other clinician, other factors influencing image quality may not be captured by anatomical detection alone. Similarly, while determining image view quality using a trained frame score classifier can incorporate factors affecting image quality beyond anatomical landmarks, this direct classification is less interpretable compared to determining image quality based on detected anatomical landmarks. Accordingly, by combining the detector-based quality score and the classifier-based quality score, the benefits of these two approaches are combined and the limitations of each approach are overcome. This provides a significant technological advantage to the ultrasound imaging analysis system 200.

Returning to method 100 in Fig. 1, at step 160 of the method the system provides the generated image view quality score for one or more of the plurality of ultrasound frames to a user via a user interface of the ultrasound imaging analysis system 200. According to an embodiment, the generated image view quality score is provided in real-time for each frame, such as when the ultrasound imaging is received or obtained. The provided output can be any of the information as described or otherwise envisioned herein, including but not limited to the generated image view quality score. The system may provide the information to a user via any mechanism, including but not limited to a visual display, an audible notification, a page, or any other method of notification. The information may be communicated by wired and/or wireless communication to another device. For example, the system may communicate the information to a mobile phone, computer, laptop, wearable device, and/or any other device configured to allow display and/or other communication of the information.

According to an embodiment, during an ultrasound scan, the system generates a quality score for each image frame, with a higher score indicating better view quality and a lower score indicating poorer quality. The score may be presented numerically (e.g., on a scale from 0 to 1), visually (e.g., with a green indicator for high-quality images and a red indicator for low-quality images), or in any other method or mechanism of providing the quality information.

At step 170 of the method, the ultrasound imaging analysis system 200 generates an overall ultrasound image view quality score, for the obtained ultrasound imaging (i.e., the cineloop) from the quality scores for a plurality of ultrasound frames. According to an embodiment, when a cineloop is saved, the confidence scores for individual frames (*c_{f}*) are combined to produce an overall cineloop-level quality score (*c_{cineloop}*). Preferably this is done by simply summing or averaging the frame-level confidences. Alternatively, frame-level confidences may be combined using a separate machine learning model.

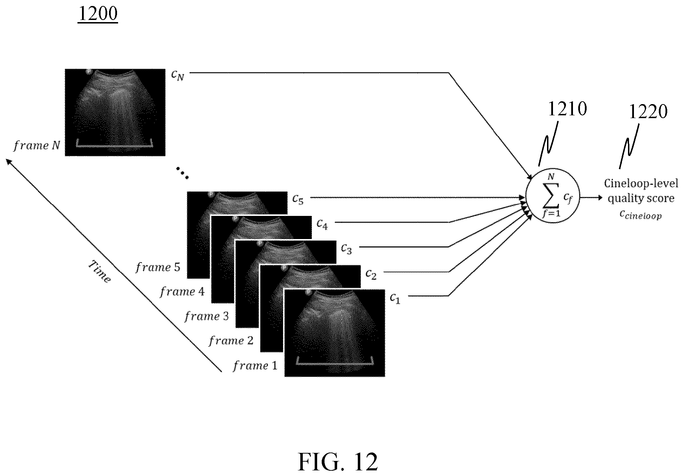

Referring to Fig. 12, in one embodiment, is a method 1200 of combining frame-level confidence scores (*c_{f}*) into an overall cineloop-level quality score (*c_{cineloop}*). The confidence score (*c_{N}*) from each frame *N* is combined at 1210, such as by summing although other methods are possible, into an overall cineloop-level quality score (*c_{cineloop}*) 1220. According to an embodiment, the confidence score (*c_{N}*) from every frame *N* is combined at 1210, while according to another embodiment a subset of frames - and the respective confidence scores (*c_{N}*) - are combined at 1210. For example, the system may sample less than all of every frame in order to generate the overall cineloop-level quality score (*c_{cineloop}*) 1220.

At step 180 of the method, the ultrasound imaging analysis system 200 provides the generated ultrasound image view quality score (e.g., the overall cineloop-level quality score (*c_{cineloop}*)) to a user via a user interface of the ultrasound imaging analysis system 200. According to an embodiment, the provided output can be any of the information as described or otherwise envisioned herein, including but not limited to the generated imaging quality score. The system may provide the information to a user via any mechanism, including but not limited to a visual display, an audible notification, a page, or any other method of notification. The information may be communicated by wired and/or wireless communication to another device. For example, the system may communicate the information to a mobile phone, computer, laptop, wearable device, and/or any other device configured to allow display and/or other communication of the information.

According to an embodiment, the ultrasound imaging analysis system 200 comprises one or more trained machine learning algorithms. For example, one or more of the anatomical landmark detection algorithm, the detector-based classification model (also called the frame classifier), and the cineloop-level quality score algorithm may be one or more trained machine learning algorithms. According to an embodiment, the anatomical landmark detection algorithm (or anatomical landmark detection branch of a joint trained machine learning algorithm) is trained to utilize input (e.g., received or obtained ultrasound imaging) to generate output (e.g., a detector-based quality score which may comprise one or more of a confidence score (*cᵢ*) - which may or may not be weighted - and location coordinates (*xᵢ, yᵢ*))*.* According to an embodiment, the frame score classifier (or frame classifier branch of a joint trained machine learning algorithm) is trained to utilize input (e.g., received or obtained ultrasound imaging) to generate output (e.g., a classifier-based quality score). According to an embodiment, the cineloop-level quality score algorithm is trained to utilize input (e.g., the detector-based quality scores for each of a plurality of ultrasound frames) to generate output (e.g., an overall cineloop-level quality score (*c_{cineloop}*)).

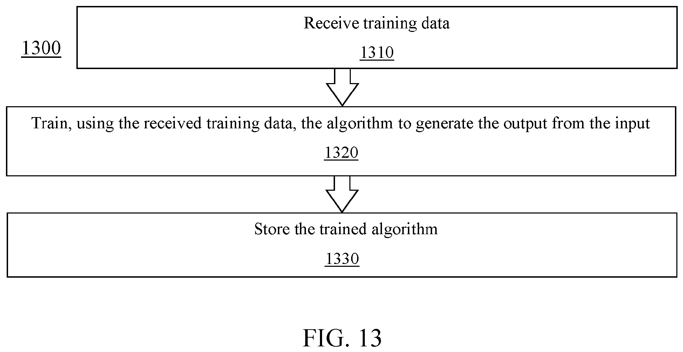

Referring to Fig. 13, in one embodiment, is a flowchart of a method 1300 for training a machine learning algorithm of the ultrasound imaging analysis system 200. The method may be performed by the ultrasound system, or may be performed by another system such as a specialized machine learning model training system. According to one embodiment, the machine learning algorithm is trained using a supervised or unsupervised training process.

At step 1310 of the method, the training system receives training data which will be used to train the model. The training data can be any data sufficient to train the model to utilize the described input data to generate the described output. For example, the training data may comprise ultrasound imaging. This training data, which could be utilized in a supervised or unsupervised manner, can comprise imaging for 100s or 1000s of patients and/or procedures, and can be updated with new imaging. The training data may also comprise other information, such as patient information. This training data may be curated by an expert such as a clinician, or it may be obtained and utilized without curation. The training data may be received from any source. For example, the training data may be received from an electronic medical record database or system, or any other component of the system or a training system. According to an embodiment, system 200 comprises or is in direct or indirect communication with an imaging database which comprises some or all of the training data set.

According to an embodiment, the training system may comprise a data pre-processor or similar component or algorithm configured to process the received training data. For example, the data pre-processor analyzes the training data to remove noise, bias, errors, and other potential issues. The data pre-processor may also analyze the input data to remove low quality data. Many other forms of data preprocessing or data point identification and/or extraction are possible.

At step 1320 of the method, the training system trains the machine learning algorithm, using the training data, to generate the respective output data from input data. The algorithm is trained using any method for training such a model. The trained algorithm is a unique model based on the training data used to train the model. Following training, the system comprises a trained machine learning algorithm. Thus, following training, the algorithm is a specialized model configured to receive the specialized input (described above) and generate the very specific output (also described above).

At step 1330 of the method, the trained algorithm is stored for future use. According to an embodiment, the trained algorithm may be stored in local or remote storage.

Referring again to Fig. 2 is a schematic representation of an ultrasound imaging analysis system 200. System 200 may be any of the systems described or otherwise envisioned herein, and may comprise any of the components described or otherwise envisioned herein. It will be understood that Fig. 2 constitutes, in some respects, an abstraction and that the actual organization of the components of the system 200 may be different and more complex than illustrated.

According to an embodiment, system 200 comprises a processor 220 capable of executing instructions stored in memory 230 or storage 260 or otherwise processing data to, for example, perform one or more steps of the method. Processor 220 may be formed of one or multiple modules. Processor 220 may take any suitable form, including but not limited to a microprocessor, microcontroller, multiple microcontrollers, circuitry, field programmable gate array (FPGA), application-specific integrated circuit (ASIC), a single processor, or plural processors.

Memory 230 can take any suitable form, including a non-volatile memory and/or RAM. The memory 230 may include various memories such as, for example L1, L2, or L3 cache or system memory. As such, the memory 230 may include static random access memory (SRAM), dynamic RAM (DRAM), flash memory, read only memory (ROM), or other similar memory devices. The memory can store, among other things, an operating system. The RAM is used by the processor for the temporary storage of data. According to an embodiment, an operating system may contain code which, when executed by the processor, controls operation of one or more components of system 200. It will be apparent that, in embodiments where the processor implements one or more of the functions described herein in hardware, the software described as corresponding to such functionality in other embodiments may be omitted.

User interface 240 may include one or more devices for enabling communication with a user. The user interface can be any device or system that allows information to be conveyed and/or received, and may include a display, a mouse, and/or a keyboard for receiving user commands. In some embodiments, user interface 240 may include a command line interface or graphical user interface that may be presented to a remote terminal via communication interface 250. The user interface may be located with one or more other components of the system, or may located remote from the system and in communication via a wired and/or wireless communications network.

Communication interface 250 may include one or more devices for enabling communication with other hardware devices. For example, communication interface 250 may include a network interface card (NIC) configured to communicate according to the Ethernet protocol. Additionally, communication interface 250 may implement a TCP/IP stack for communication according to the TCP/IP protocols. Various alternative or additional hardware or configurations for communication interface 250 will be apparent.

Storage 260 may include one or more machine-readable storage media such as read-only memory (ROM), random-access memory (RAM), magnetic disk storage media, optical storage media, flash-memory devices, or similar storage media. In various embodiments, storage 260 may store instructions for execution by processor 220 or data upon which processor 220 may operate. For example, storage 260 may store an operating system 261 for controlling various operations of system 200.

It will be apparent that various information described as stored in storage 260 may be additionally or alternatively stored in memory 230. In this respect, memory 230 may also be considered to constitute a storage device and storage 260 may be considered a memory. Various other arrangements will be apparent. Further, memory 230 and storage 260 may both be considered to be non-transitory machine-readable media. As used herein, the term non-transitory will be understood to exclude transitory signals but to include all forms of storage, including both volatile and non-volatile memories.

While system 200 is shown as including one of each described component, the various components may be duplicated in various embodiments. For example, processor 220 may include multiple microprocessors that are configured to independently execute the methods described herein or are configured to perform steps or subroutines of the methods described herein such that the multiple processors cooperate to achieve the functionality described herein. Further, where one or more components of system 200 is implemented in a cloud computing system, the various hardware components may belong to separate physical systems. For example, processor 220 may include a first processor in a first server and a second processor in a second server. Many other variations and configurations are possible.

According to an embodiment, system 200 comprises or is in direct or indirect communication with an imaging modality 270. The imaging modality can be any modality sufficient to obtain imagery utilized by the ultrasound system 200 to obtain ultrasound imagery. The imaging modality can be, for example, an ultrasound system such as EPIQ^{®} system, Philips Lumify^{®}, and Philips Affinity^{®}, among many others.

According to an embodiment, system 200 comprises or is in direct or indirect communication with an image database 280. The image database may be any image database, and may comprise images or videos or reports or other data obtained using any imaging modality, such as imaging modality 270. The image database 280 may be local to the ultrasound system, and may optionally be a component of the system. The image database 280 may alternatively be remote to the ultrasound system.

According to an embodiment, storage 260 of system 200 may store one or more algorithms, modules, and/or instructions to carry out one or more functions or steps of the methods described or otherwise envisioned herein. For example, storage 260 may comprise, among other instructions or data, an anatomical landmark detection algorithm 262, a detector-based classification model (also called the frame classifier) 263, a cineloop-level quality score algorithm 264, and/or reporting instructions 265.

According to an embodiment, anatomical landmark detection algorithm 262 of the ultrasound imaging analysis system 200 is trained to analyze the received input, including but not limited to received or obtained ultrasound imaging (as input to the model) to generate a detector-based quality score which may comprise one or more of a confidence score (*cᵢ*) - which may or may not be weighted - and location coordinates (*xᵢ, yᵢ*) (as output of the model). The anatomical landmark detection algorithm can be any model that can be trained to utilize the input to generate the output, as described or otherwise envisioned herein. According to one possible embodiment, the anatomical landmark detection algorithm can be a neural network or other trained machine learning model, for example a convolutional neural network (CNN) or a transformer network or other neural network. According to another embodiment, the anatomical landmark detection algorithm is a traditional algorithm, utilizing programming to analyze the input (a predefined set of rules or procedures to perform a specific task).

According to an embodiment, frame classifier 263 of the ultrasound imaging analysis system 200 is trained to analyze the received input, including but not limited to received or obtained ultrasound imaging (as input to the model) to generate a classifier-based quality score (as output of the model). The frame classifier can be any model that can be trained to utilize the input to generate the output, as described or otherwise envisioned herein. According to one possible embodiment, the frame classifier can be a neural network or other trained machine learning model, for example a convolutional neural network (CNN) or a transformer network or other neural network. According to another embodiment, the frame classifier is a traditional algorithm, utilizing programming to analyze the input (a predefined set of rules or procedures to perform a specific task).

According to an embodiment, cineloop-level quality score algorithm 264 of the ultrasound imaging analysis system 200 is trained to analyze the received input, including but not limited to the detector-based quality scores for each of a plurality of ultrasound frames (as input to the model) to generate an overall cineloop-level quality score (*c_{cineloop}*) (as output of the model). The cineloop-level quality score algorithm can be any model that can be trained to utilize the input to generate the output, as described or otherwise envisioned herein. According to one possible embodiment, the cineloop-level quality score algorithm can be a neural network or other trained machine learning model, for example a convolutional neural network (CNN) or a transformer network or other neural network. According to another embodiment, the cineloop-level quality score algorithm is a traditional algorithm, utilizing programming to analyze the input (a predefined set of rules or procedures to perform a specific task).

According to an embodiment, reporting instructions 265 direct the system to provide the output of the system to a user, such as a clinician, via a user interface. The provided output can be any of the information as described or otherwise envisioned herein, including but not limited to any of the scores generated by the system, such as the generated image view quality score for one or more of the plurality of ultrasound frames, and/or the overall cineloop-level quality score (*c_{cineloop}*). According to an embodiment, the generated image view quality score is provided in real-time for each frame, such as when the ultrasound imaging is received or obtained. The system may provide the information to a user via any mechanism, including but not limited to a visual display, an audible notification, a page, or any other method of notification. The information may be communicated by wired and/or wireless communication to another device. For example, the system may communicate the information to a mobile phone, computer, laptop, wearable device, and/or any other device configured to allow display and/or other communication of the information.

According to an embodiment, the ultrasound imaging analysis system 200 is configured to process many thousands or millions of datapoints in input data used to train one or more of the machine learning algorithms. For example, generating a functional and skilled trained algorithm from a corpus of training data requires processing of millions of datapoints from input data and generated features. This can require millions or billions of calculations to generate a novel trained algorithm from those millions of datapoints and millions or billions of calculations. As a result, each trained algorithm is novel and distinct based on the input data and parameters of the model, and thus improves the functioning of the system. Generating a functional and skilled trained algorithm comprises a process with a volume of calculation and analysis that a human brain cannot accomplish in a lifetime, or multiple lifetimes.

Additionally, the ultrasound imaging analysis system 200 is configured to process many thousands or millions of datapoints in the ultrasound imaging in order to generate the output information, including but not limited to one or more of the generated image view quality score for one or more of the plurality of ultrasound frames, and/or the overall cineloop-level quality score. This comprises a volume of calculation and analysis that a human brain cannot accomplish in a lifetime, or multiple lifetimes. Further, the real-time nature of the claimed method and system also comprises an analysis that a human brain cannot accomplish with or without pen/pencil or paper. For example, ultrasound imaging can comprise many frames per second (fps), ranging from a low of 10-30 fps to a high of 50-100 fps (and sometimes even higher such as for echocardiography). The human mind is unlikely to be able to analyze the quality of a single ultrasound frame in one second, much less 10 or 100 frames in one second. Thus, the human mind alone cannot analyze the quality of frames in real-time, much less communicate that quality in real-time. as a result of the real-time display of image quality, as described herein, the ultrasound imaging analysis system 200 comprises a significant technological advancement over existing ultrasound systems.

All definitions, as defined and used herein, should be understood to control over dictionary definitions, definitions in documents incorporated by reference, and/or ordinary meanings of the defined terms.

The indefinite articles "a" and "an," as used herein in the specification and in the claims, unless clearly indicated to the contrary, should be understood to mean "at least one."

The phrase "and/or," as used herein in the specification and in the claims, should be understood to mean "either or both" of the elements so conjoined, i.e., elements that are conjunctively present in some cases and disjunctively present in other cases. Multiple elements listed with "and/or" should be construed in the same fashion, i.e., "one or more" of the elements so conjoined. Other elements may optionally be present other than the elements specifically identified by the "and/or" clause, whether related or unrelated to those elements specifically identified.

As used herein in the specification and in the claims, "or" should be understood to have the same meaning as "and/or" as defined above. For example, when separating items in a list, "or" or "and/or" shall be interpreted as being inclusive, i.e., the inclusion of at least one, but also including more than one, of a number or list of elements, and, optionally, additional unlisted items. Only terms clearly indicated to the contrary, such as "only one of" or "exactly one of," or, when used in the claims, "consisting of," will refer to the inclusion of exactly one element of a number or list of elements. In general, the term "or" as used herein shall only be interpreted as indicating exclusive alternatives (i.e. "one or the other but not both") when preceded by terms of exclusivity, such as "either," "one of," "only one of," or "exactly one of."

As used herein in the specification and in the claims, the phrase "at least one," in reference to a list of one or more elements, should be understood to mean at least one element selected from any one or more of the elements in the list of elements, but not necessarily including at least one of each and every element specifically listed within the list of elements and not excluding any combinations of elements in the list of elements. This definition also allows that elements may optionally be present other than the elements specifically identified within the list of elements to which the phrase "at least one" refers, whether related or unrelated to those elements specifically identified.

It should also be understood that, unless clearly indicated to the contrary, in any methods claimed herein that include more than one step or act, the order of the steps or acts of the method is not necessarily limited to the order in which the steps or acts of the method are recited.

In the claims, as well as in the specification above, all transitional phrases such as "comprising," "including," "carrying," "having," "containing," "involving," "holding," "composed of," and the like are to be understood to be open-ended, i.e., to mean including but not limited to. Only the transitional phrases "consisting of" and "consisting essentially of" shall be closed or semi-closed transitional phrases, respectively.

While several inventive embodiments have been described and illustrated herein, those of ordinary skill in the art will readily envision a variety of other means and/or structures for performing the function and/or obtaining the results and/or one or more of the advantages described herein, and each of such variations and/or modifications is deemed to be within the scope of the inventive embodiments described herein. More generally, those skilled in the art will readily appreciate that all parameters, dimensions, materials, and configurations described herein are meant to be exemplary and that the actual parameters, dimensions, materials, and/or configurations will depend upon the specific application or applications for which the inventive teachings is/are used. Those skilled in the art will recognize, or be able to ascertain using no more than routine experimentation, many equivalents to the specific inventive embodiments described herein. It is, therefore, to be understood that the foregoing embodiments are presented by way of example only and that, within the scope of the appended claims and equivalents thereto, inventive embodiments may be practiced otherwise than as specifically described and claimed. Inventive embodiments of the present disclosure are directed to each individual feature, system, article, material, kit, and/or method described herein. In addition, any combination of two or more such features, systems, articles, materials, kits, and/or methods, if such features, systems, articles, materials, kits, and/or methods are not mutually inconsistent, is included within the inventive scope of the present disclosure.

## Claims

1. A method (100) for analyzing quality of ultrasound imaging, comprising:
receiving (120) ultrasound imaging obtained from a first location of a patient, the ultrasound imaging comprising a plurality of ultrasound frames per second;
analyzing (130) the received ultrasound imaging with an anatomical landmark detection algorithm to identify one or more anatomical landmarks in the received ultrasound imaging, wherein a detector-based quality score is determined based on the analysis by the anatomical landmark detection algorithm, the detector-based quality score comprising a likelihood of the anatomical landmark's presence in the received ultrasound imaging;
analyzing (140) the received ultrasound imaging with a trained frame score classifier to generate a classifier-based quality score for each of the plurality of ultrasound frames, wherein the classifier-based quality score indicates a quality of a respective ultrasound frame;
generating (150), by combining the detector-based quality score and the classifier-based quality score, an image view quality score for each of the plurality of ultrasound frames;
providing (160), to a user via a user interface, the generated image view quality score for one or more of the plurality of ultrasound frames; and
generating (170), from the generated image view quality scores for a plurality of ultrasound frames, an ultrasound image view quality score.

2. The method of claim 1, wherein the generated image view quality score is a sum or average of quality scores for each of a plurality of anatomical landmarks in the received ultrasound imaging.

3. The method of claim 1, wherein the generated image view quality score comprises a weight indicating an importance of the presence of the respective anatomical landmark in the received ultrasound imaging.

4. The method of claim 1, wherein the anatomical landmark detection algorithm is a trained machine learning model.

5. The method of claim 1, wherein the anatomical landmark detection algorithm and the trained frame score classifier are a single model.

6. The method of claim 1, wherein the classifier-based quality score is a value including or between 0 and 1.

7. The method of claim 1, wherein the anatomical landmark detection algorithm and the trained frame score classifier analyze the received ultrasound imaging in sequence.

8. The method of claim 1, wherein the anatomical landmark detection algorithm and the trained frame score classifier analyze the received ultrasound imaging in parallel.

9. The method of claim 1, wherein the generated image view quality score is provided via the user interface as a numerical score or as a color.

10. The method of claim 1, wherein the ultrasound imaging quality score is generated by a trained ultrasound imaging quality score model.

11. The method of claim 1, further comprising the step of providing (180), to a user via a user interface, the generated ultrasound imaging quality score.

12. A system (200) for analyzing quality of ultrasound imaging, comprising:
an anatomical landmark detection algorithm (262) configured to analyze received ultrasound imaging to identify one or more anatomical landmarks in the received ultrasound imaging, wherein a detector-based quality score is determined based on the analysis by the anatomical landmark detection algorithm, the detector-based quality score comprising a likelihood of the anatomical landmark's presence in the received ultrasound imaging;
a trained frame score classifier (263) configured to analyze received ultrasound imaging to generate a classifier-based quality score for each of the plurality of ultrasound frames, wherein the classifier-based quality score indicates a quality of a respective ultrasound frame;
a user interface (240); and
a processor (220) configured to: (i) receive ultrasound imaging obtained from a first location of a patient, the ultrasound imaging comprising a plurality of ultrasound frames per second; (ii) analyze the received ultrasound imaging with the anatomical landmark detection algorithm to generate a detector-based quality score for each of the plurality of ultrasound frames; (iii) analyze the received ultrasound imaging with the trained frame score classifier to generate a classifier-based quality score for each of the plurality of ultrasound frames; (iv) combine the detector-based quality score and the classifier-based quality score to generate an image view quality score for each of the plurality of ultrasound frames; (v) provide, via the user interface, the generated image view quality score for one or more of the plurality of ultrasound frames; and (vi) generate an ultrasound image view quality score from the generated image view quality scores for a plurality of ultrasound frames.

13. The system of claim 12, wherein the anatomical landmark detection algorithm and the trained frame score classifier are a single model.

14. The system of claim 12, wherein the generated image view quality score is provided via the user interface as a numerical score or as a color.

15. The system of claim 12, wherein the processor is further configured to provide, to a user via the user interface, the generated ultrasound imaging quality score.
